(19) Europäisches Patentamt
European Patent Office
Office européen des brevets

(11) Publication number: **0 413 299 A1**

# (12) EUROPEAN PATENT APPLICATION

(21) Application number: **90115553.1**

(22) Date of filing: **14.08.90**

(51) Int. Cl.5: **A61K 31/71**, A61K 9/16, A61K 9/18, A61K 9/20, A61K 9/48, A61K 9/50

(30) Priority: **16.08.89 JP 211002/89**

(43) Date of publication of application: **20.02.91 Bulletin 91/08**

(84) Designated Contracting States: **DE ES FR GB IT**

(71) Applicant: **MERCIAN CORPORATION**
**5-8, Kyobashi 1-chome**
**Chuo-ku, Tokyo(JP)**

(72) Inventor: **Nishimura, Masami**
**7-4-D-108, Ichinomiya, Samukawa-cho**
**Koza-gun, Kanagawa-ken(JP)**
Inventor: **Yamaguchi, Toshio**
**3-10-5, Honcho**
**Fujisawa-shi, Kanagawa-ken(JP)**
Inventor: **Tone, Hiroshi**
**3-7-4-1003 Namiki, Kanazawa-ku**
**Yokohama-shi, Kanagawa-ken(JP)**
Inventor: **Okamoto, Rokuro**
**2-18, Hanaoki**
**Fujisawa-shi, Kanagawa-ken(JP)**
Inventor: **Takeuchi, Tomio**
**5-1-11, Higashigotanda, Shinagawa-ku**
**Tokyo(JP)**

(74) Representative: **von Kreisler, Alek, Dipl.-Chem. et al**
**Patentanwälte Von Kreisler-Selting-Werner,**
**Deichmannhaus am Hauptbahnhof**
**D-5000 Köln 1(DE)**

## (54) Antibiotic composition.

(57) An antibiotic composition comprising a dispersoid of a substantially non-crystalline 4″-O-(para-methoxyphenylacethyl)tylosin antibiotic dispersed in a base material selected from the group consisting of acrylic copolymers and cellulose polymer derivatives is disclosed. Also disclosed are a method for preparation of an antibiotic composition, a pharmaceutical composition for the treatment of an infectious disease, and a method for the treatment of an infectious disease by administering an antibiotic composition.

EP 0 413 299 A1

# ANTIBIOTIC COMPOSITION

## BACKGROUND OF THE INVENTION

### Field of the Invention

The present invention relates to a composition comprising an antibiotic useful for teh treatment of bacterial infectious diseases. More specifically, the present invention relates to an antibiotic composition comprising a macrolide antibiotic having an excellent water solubility and an improved delivery when administered orally.

### Description of the Prior Art

4″-O-(Para-methoxyphenylacetyl)tylosin is a macrolide antibiotic represented by the following formula:

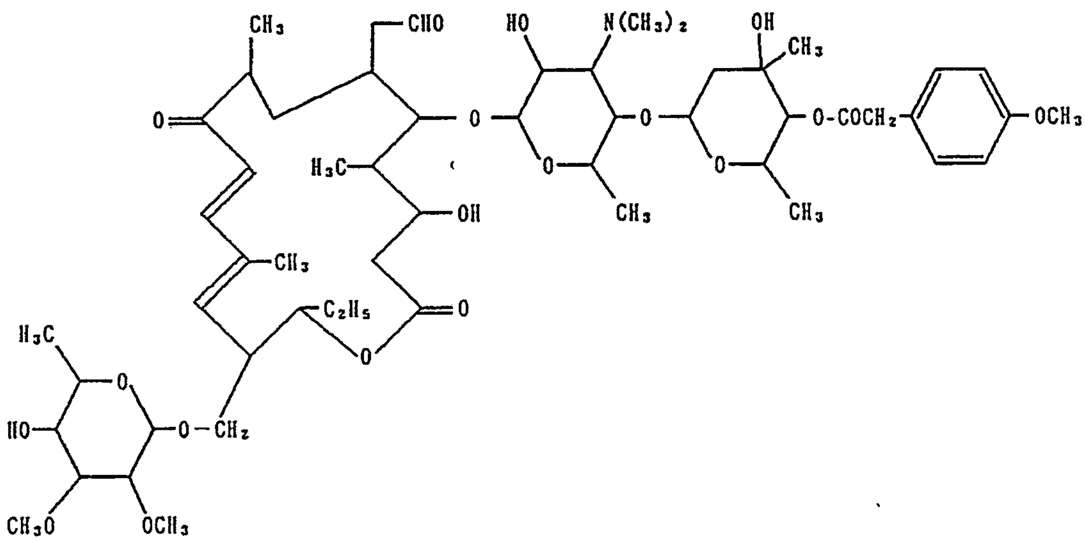

which is an analog of tylosin having a 16 membered ring structure. Japanese Patent Unexamined Publication No. 137895/1986 discloses that the above antibiotic has an excellent antibacterial activity against gram-negative and gram-positive bacteria, and is suitable for oral administration.

However, this antibiotic, like other macrolide antibiotics, is only very slightly soluble in water. Since the antibiotic, per se, does not readily dissolve in the gastric or intestinal juices when administered orally, it is not fully absorbable from the intestines, which results in insufficient treatment of the infectious diseases. For example, the solubility of the antibiotic in a crystalline state is 1200μg/ml in an acidic aqueous solution of pH 2, however, the solubility in an acidic solution decreases remarkably as the pH of the solution becomes higher pH: The solubilities of the antibiotic in acidic solutions of pH 3, 4, 5, and 6.8 are 150, 10, 2 and 0.5μg/ml, respectively.

The pH value of the gastric juices of a healthy person is about 1.2, however, the values increase as people reach middle or advanced ages. It has been revealed that about 35% of those in their 40s and about not less than 60% of those in their 50s are classified as hypochlorhydria or achlorhydria (J. Pharm. Dyn. 7: 656, 1984). As a consequence, the absorption of the above antibiotic differs from individual to individual, and thus the clinical treatment may be significantly affected by the delivery of said antibiotic for each patient.

A method involving the elimination of crystalline state of the above antibiotic in order to overcome water solubility problems is disclosed at pages 50 - 52 of Iyakuhin-Kaihatsu-Kiso-Kouza No. 16 (method for designing a pharmaceutical composition II). If the disclosed method is applied to the above antibiotic to obtain a composition comprising the non-crystalline state antibiotic by, for example, spray drying the solution containing said antibiotic, or by fluidized bed granulation using additives such as avicell, lactose, or

starch as a nucleus for granulation, the obtained composition becomes well soluble in water. However, the antibiotic immediately changes to the crystalline state after dispersion of the composition in water, which results in a significant decrease of the solubility of said antibiotic in water: Thus the solubility of the antibiotic is not successfully increased. Such a transition from non-crystalline state to crystalline state may occur during the storage of the non-crystallized powder, and the powder will become insoluble in proportion to increasing length of storage. For example, a time course investigation of the solubility in water at pH 4 of the non-crystallized product obtained according to the above method showed that the solubility was 770 µg/ml after 5 min., however, it decreased to become 660, 500, 280 and 150 µg/ml (37° C) after 10, 15, 30, and 60 minutes, respectively. Such decrease in solubility was believed to be caused by the transition of non-crystalline particles to crystals in water since the remarkable polarized light was detectable in the precipitates as examined under a polarizing microscope. In addition, if the above-mentioned non-crystallized product was stored at 60° C under airtight conditions, the polarization of the product gradually increased after seven days of storage. The activation energy ( $\Delta$ H mcal/mg) calculated by the area of the exothermic peak at the crystalline transition temperature (Tc: 130 ° C) measured by differential scanning thermal analysis (DSC) was 12.8 at the beggining of storage, however, it decreased with time to become 11.4, 9.6, and 5.0 after 7, 14, and 28 days, respectively. The solubility (maximum) at pH 4.0 decreased from 770µg/ml to 690, 510, and 240 µg/ml (37° C) oorreponding to the decrease of the activation energy. Further, the method for stabilizing the non-crystalline product comprising the step of dispersing the non-crystallized antibiotic in a pharmaceutical composition comprising a base material such as ethylcellulose (Chem. Pharm. Bull. 29(9), 2675-2682, 1981) failed to provide a sufficiently soluble and stable composition.

## SUMMARY OF THE INVENTION

An object of the present invention is therefore to provide a stable antibiotic composition comprising 4″-O-(para-methoxyphenylacetyl)tylosin antibiotic, which shows excellent solubility in water and delivery as administered orally.

Another object of the present invention is to provide a method for preparing the antibiotic composition comprising 4″-O-(para-methoxyphenylacetyl)tylosin antibiotic.

A further object of the present invention is to provide a pharmaceutical composition for the treatment of an infectious disease comprising 4″-O-(para-methoxyphenylacetyl)tylosin antibiotic together with a pharmaceutically acceptable carrier or coating.

The inventors of the present invention have conducted various studies to achieve the foregoing objects and found that an antibiotic composition, prepared by dispersing said antibiotic in substantially non-crystalline state in a base material selected from the group consisting of acrylic copolymers and cellulose polymer derivatives, has improved water solubility and delivery and is stable after prolonged storage. The objects of the present invention can thus be effectively attained by providing such an antibiotic composition.

In accordance with the above object, the present invention provides an antibiotic composition comprising a dispersoid of a substantially non-crystalline 4″-O-(para-methoxyphenylacetyl)tylosin antibiotic dispersed in a base material selected from the group consisting of acrylic copolymers and cellulose polymer derivatives.

In accordance with another embodiment of the present invention, a method is provided for preparing an antibiotic composition comprising a dispersoid of a substantially non-crystalline 4″-O-(para-methoxyphenylacetyl)tylosin antibiotic dispersed in a base material selected from the group consisting of acrylic copolymers and cellulose polymer derivatives, comprising the steps of dissolving the 4″-O-(para-methoxyphenylacetyl)tylosin antibiotic and the base material in a solvent, and removing the solvent.

In accordance with yet another embodiment of the present invention, a pharmaceutical composition is provided for the treatment of an infectious disease comprising an effective amount of a dispersoid of a substantially non-crystalline 4″-O-(para-methoxyphenylacetyl)tylosin antibiotic dispersed in a base material selected from the group consisting of acrylic copolymers and cellulose polymer derivatives together with a pharmaceutically acceptable carrier or coating.

In accordance with a further embodiment, the present invention provides a method for the treatment of an infectious disease comprising the step of administering to a mammal an effective amount of an antibiotic composition comprising a dispersoid of a substantially non-crystalline 4″-O-(para-methoxyphenylacetyl)-tylosin antibiotic dispersed in a base material selected from the group consisting of acrylic copolymers and cellulose polymer derivatives together with a pharmaceutically acceptable carrier or coating.

Further objects, features and advantages of the present invention will become apparent from the Description of the Preferred Embodiments which follows, when read in light of the attached Examples.

## BRIEF DESCRIPTION OF THE DRAWINGS

In the drawings:

Fig. 1 shows the X-ray diffraction patterns of the antibiotic composition of the present invention;
Fig. 2 shows the solubility with time of the capsules of Example 2;
Fig. 3 shows the solubility with time of the tablets of Examples 3 and 4;
Fig. 4 shows the solubility with time of the granules of Example 5;
Fig. 5 shows the solubility with time of the granules of Example 5;
Fig. 6 shows the solubitity with time of the tablets of Example 6; and
Fig. 7 shows the blood level of the antibiotic according to Experiment 5.

## DETAILED DESCRIPTION OF THE PREFERRED EMBODIMENT

The present invention provides an antibiotic composition comprising a dispersoid of a substantially non-crystalline 4″-O-(para-methoxyphenylacetyl)tylosin antibiotic dispersed in a base material selected from the group consisting of acrylic ccpolymers and cellulose polymer derivatives and a method for preparing the composition. The present invention also provides a pharmaceutical composition comprising an effective amount of a dispersoid of a substantially non-crystalline 4″-O-(para-methoxyphenylacetyl)tylosin antibiotic dispersed in a base material selected from the group consisting of acrylic copolymers and cellulose polymer derivatives.

Japanese Unexamined Patent Publication No. 137895/1986 discloses 4″-O-(Para-methoxyphenylacetyl)-tylosin, a macrolide antibiotic, as Example 10, and the antibiotic may be prepared according to the method described in the prior art example. The antibiotic prepared by the above-mentioned method is isolated in crystalline state and usually has no water of crystallization. The solubility of the antibiotic in crystalline state is 1200, 50, 10, 2, and 0.5μg/ml (37° C in the aqueous acidic solution of pH 2, 3, 4, 5, and 6.8, respectively.

According to the present invention, the antibiotic is dispersed in a substantially non-crystalline state in a base material selected from the group consisting of acrylic copolymers and cellulose polymer derivatives.

Examples of the acrylic copolymers include methacrylic acid/methacrylate copolymers, methacrylate/methacrylate copolymers, methacrylic acid/methacrylate/aminoalkylmethacrylate copolymers, methacrylate/methacrylate/aminoalkylmethacrylate copolymers, and mixtures thereof. Preferred acrylic coplymers include aminoalkylmethacrylate copolymer E (methyl methacrylate/butyl methacrylate/dimethylaminoethyl methacrylate copolymer). For example, Eudragit E 100 copolymer (Rhorm Pharma) may be used.

Examples of cellulose polymer derivatives include cellulose phthalate derivatives and cellulose acetate succinate derivatives, which generally are used as enteric coating agents for granules or tablets. For example, hydroxypropylmethylcellulose phthalate, hydroxypropylmethylcellolose acetate succinate, carboxymethylethylcel lulose, or the mixture of the above compounds may be used. Among these cellulose polymer derivatives, carboxymethylethylcellulose is preferred.

In addition, a mixture of the acrylic copolymer and the cellulose polymer derivative may also be used.

The dispersion of said antibiotic in substantially non-crystalline state in a base material selected from the group consisting of acrylic copolymers and cellulose polymer derivatives may be carried out by dissolving said antibiotic and the base material in a solvent and then removing the solvent.

After dissolving the polymer base material and said antibiotic in a solvent, any additive generally used for the manufacture of pharmaceutical compositions such as, for example, excipients, binders, disintegrators, lubricants, and the like, may be dissolved or suspended in the resulting solution.

Any solvent may be used in the above process provided that the solvent can dissolve both the antibiotic and the polymer base material, and examples of the solvent include chloroform, methylene chloride, methanol, ethanol, isopropanol, and mixture thereof.

The dispersoid comprising the dispersed antibiotic in substantially non-crystalline state may be prepared by removing a solvent from a solution containing from 0.5 to 50% by weight of the polymer base material and from 20 to 40% by weight of the antibiotic in the solvent mentioned above. The ratio of the polymer base material to the antibiotic may be from 0.02 to 3 part by weight of the polymer base material to 1 part by weight of the antibiotic, and preferably from 0.05 to 0.5 part by weight of the polymer base material to 1 part by weight of the antibiotic. If the ratio of the polymer base material to the antibiotic is lower than 0.02 part by weight to 1 part by weight, the antibiotic cannot be kept stably in the non-crystalline state and will precipitate as crystals. On the other hand, if the ratio of the polymer base material to the antibiotic is larger than 3 part by weight to 1 part by weight, the amount of the antibiotic in the resulting

dispersoid becomes too small, this results in an increased volume of the pharmaceutical composition which becomes inconvenient for oral administration of the unit dose of the compostion. In addition, the solution may preferably prepared at a temperature of from 0 to 50° C.

Any industrially available method for removing a solvent such as, for example, evaporating the solvent under a reduced pressure or an atmospheric pressure or spray drying may be used to prepare a dispersoid comprising the dispersed antibiotic in a substantially non-crystalline state in a base material by removing a solvent. Evaporation under reduced pressure may generally be carried out at a temperature of from 5 to 40 °C under a reduced pressure of about from to 10 mmHg, and the evaporation under atmospheric pressure may be carried out at a temperature of from 50 to 100° C . The spray drying process may be carried out at an inlet temperature of from 40 to 150° C and at an outlet temperature of from 10 to 90° C. After removing the solvent by one of the above-mentioned processes, the resulting dispersoid may contain not more than 0.1% by weight of the solvent, which is suitable for the use as a pharmaceutical composition.

According to the method described above, the antibiotic composition of the present invention can be prepared, comprising the dispersoid of the substantially non-crystalline antibiotic dispersed in the polymer base material: The dispersoid can usually be obtained as an uniform dispersoid in a powder form. According to the present invention, substantially non-crystalline state of the antibiotic may contain not less than 95% by weight of non-crystalline antibiotic based on the total weight of the antibiotic dispersed in the polymer base material. the noncrystalline state of the antibiotic is generally defined as an "amorphous state", which includes the solid state of a mass having a periodic arrangement of atoms in part of the mass which does not give any siginificant X-ray diffraction peaks as examined by X-ray analysis, as well as the solid state of a mass material which does not have any regular crystal lattices of atoms or ions.

The water solubility of the powdered dispersoid obtained according to the method descrive above may be not less than 200µg/ml (37° C), and the transition of the antibiotic from non-crystalline state to crystalline state will not occur after the storage for one month at 40° C and 75% relative humidity. In addition, if the dispersoid is dissolved in water, the crystallization of the antibiotic will be much delayed and, as a consequence, the solution is stable for a sufficiently long time without any precipitation of the antibiotic. Further, the antibiotic contained in the dispersoid can be well absorbed from intestines to give a high blood concentration, thus the antibiotic composition shows excellent delivery when administered orally.

The dispersoid, per se, may be administered to a patient as an antibiotic composition of the present invention in the form of powders or in the form of capsules filled with the dispersoid. Preferably, the antibiotic composition of the present invention may be administered as the pharmaceutical composition of the present invention in the form of granules, capsules, or tablets, which comprise pharmaceutically acceptable carriers or coatings for granulation. Such coatings or carriers comprise, for example, excipients, binders, surfactants, or lubricants. The above pharmaceutically acceptable carriers or coatings may be added in the amount of from 5 to 80% by weight based on the total weight of the pharmaceutical composition.

The pharmaceutical composition of the present invention containing the pharmaceutically acceptable carriers or coatings may be prepared by mixing the dispersant with the pharmaceutically acceptable carriers or coatings described above by using such an apparatus as a twin-cylinder mixer. Alternatively, the pharmaceutical composition may be obtained by removing the solvent from the mixture obtained by adding or spraying a solution containing the antibiotic and the polymer base material to or onto pharmaceutically acceptable carriers or coatings by ordinary methods under a reduced pressure or under atmospheric pressure.

The pharmaceutical composition of the present invention is suitable, for example, for oral administration or rectal administration for the treatment of an infectious disease. The unit dosage of the pharmaceutical compositon of the present invention for an adult patient may generally be from about 100 to 400 mg equivalent of the antibiotic. The pharmaceutical composition may generally be administered three times a day in a dose of from 600 to 1,200 mg per day, however, the dose may be increased or decreased depending on, for example, the types of bacteria, or the age and the conditions of the patient to be treated. Preferable unit dose ranges from 100 to 200 mg equivalent of the antibiotic. $LD_{50}$ value of the antibiotic composition of the present invention measured by an acute toxicity test using male ICR mouse was not less than 2.0 g/kg (p.o.), which shows that the antibiotic composition of the present invention has extremely low toxicity, thus is very safe even when administrered at high dosages.

From the foregoing descripition, those skilled in the art can easily understand that the antibiotic composition of the present invention is highly soluble as dispersed in water, and the non-crystalline state of the antibiotic comprised in the composition can be maintained stably during storage. As a consequence, it immediately dissolves after an oral or rectal administration, which results in a excellent absorption of the antibiotic, and thus is quite useful for the treatment of infectious diseases.

The present invention will be further illustrated by the following Examples and Reference Examples. The Examples are given by way of illustration only and are not to be construed as limiting.

## EXAMPLES

### Reference 1

4″-O-(para-methoxyphenylacetyl)tylosin was prepared by the method described in Example 10 of the Japanese Patent Unexamined Publication No. 137895/1986. The compound obtained had the following physical properties: colorless crystals; m.p. 215-230 °C (decomp.).

### Example 1

100 g of the antibiotic of Reference 1 was dissolved in 300 *ml* of methylene chloride together with 50, 20, 10, or 5g of Eudragit E 100 at 25°C . The resulting solutions were spray-dried by using a spray drying apparatus at 80 °C to obtain powdered dispersoids.

### Example 2

150 g of crystalline cellulose was placed in a fluid bed granulation drying apparatus. The nuclei of the crystalline cellulose were sprayed at 60°C with the solution previously prepared by dissolving 200 g of the above-obtained antibiotic and 40g of Eudragit E 100 in 600*ml* of methylene chloride at 25°C . After granulation, the granules were dried at 40 °C for 60 minutes, and then 100 g of carboxymethylcellulose was added to the granules and the resulting mixture was uniformly mixed. The resulting uniform composition was filled into JPIII capsules to obtain capsules.

### Example 3

96 g of crystalline cellulose and 80 g of carboxymethylcellulose were placed in a fluid bed granulation drying apparatus to obtain granules according to the method described in Example 2. A previously-prepared solution, made by dissolving 200 g of the above-obtained antibiotic and 20g of Eudragit E 100 in 600*ml* of methylene chloride at 40°C , was used. After 4.5 g of magnesium stearate was added to the granules and then mixed, the resulting composition was compressed to obtain tablets of 10 mm in diameter (200 mg equivalent/tablet).

### Example 4

20 g of crystalline cellulose, 80 g of carboxymethylcellulose, 40 g of corn starch, and 20 g of carboxymethylethylcellulose were placed in a fluid bed granulation drying apparatus to obtain granules according to the method described in Example 2. A previously-prepared solution, made by dissolving 200 g of the above-obtained antibiotic and 20g of Eudragit E 100 in 600*ml* of methylene chloride at 40°C, was used. After 8 g of magnesium stearate was added to the granules and then mixed, the resulting composition was compressed to obtain tablets of 10 mm in diameter (200 mg equivalent/tablet).

### Example 5

100 g of the above-obtained antibiotic was dissolved in 300 ml of the mixture of methylene chloride and ethanol (1:1) together with 50, 20, 10, or 5g of carboxymethylethylcellulose at 25°C , and the resulting solutions were spray-dried at 80°C to obtain powders. After 100g of crystalline cellulose and 2g of magnesium stearate were added to the powders and then mixed uniformly, the composition obtained was granulated by using a dry granulator, followed by adjusting the grain size to obtain 24-80 mesh granules.

Example 6

120 g of crystalline cellulose and 80 g of carboxymethylcellulose were placed in a fluid bed granulation drying apparatus to obtain granules according to the method described in Example 2 by using the solution previously prepared by dissolving 200 g of the above- obtained antibiotic and 20 g of carboxymethylethylcellulose in 600 ml of the mixture of methylene chloride and ethanol (1:1) at 30° C. After 4 g of magnesium stearate was added to the granules, the resulting composition was compressed to obtain tablets of 10 mm in diameter (200 mg equivalent/tablet).

Stabilities of the above pharmaceutical compositions at 60° C for one month under airtight conditions or at 40 °C for one month under 75% RH, and blood concentration of the antibiotic after administration of the above pharmaceutical compositions were determined as follows:

Experiment 1

Examination under a polarizing microscope

Polarization of the above pharmaceutical composition was examined under a polarizing microscope (Olympus, BH-2) while rotating sample stage. As shown in Table 1, no polarization was observed with samples of Examples 1 and 5, and samples of References 1 and 3 soon after the preparation of the samples. Polarization was observed after one month storage at 60 °C or 40°C/75 %RH with samples of Reference 1 and Reference 3 which do not contain Eudragit E 100 and carboxymethylethylcellulose, respectively. On the other hand, no polarization was observed with samples of Example 1 and 5 which contain Eudragit E 100 and carboxymethylethylcellulose, respectively.

Experiment 2

Determination of crystal transition energy (ΔH) by DSC method

The crystal transition energies of the above samples were determined by using a Micro DSC standard-type apparatus (Rigaku Corporation). About 7mg (potency equivalent amount) of sample powders were placed on the aluminum sampling pan and were heated at the rate of 10 °C /min. The area of the exothermic peak appearing at about 130 °C was calucurated by half width method to obtain the activation energy ( Δ H : mcal/mg) accompanied with the transition to crystalline state. As shown in Table 1, the values of Δ H of the samples of Refereces 1, 2, and 3 were about from 13 to 15 soon after the preparation of the samples, while the values ofΔ H decreased after one month storage at 60°C or at 40 °C/75 %RH. The results reveal that the transition to a crystalline state occured with the Reference samples, as supported by the polarization results obtained by Experiment 1. On the other hand, the values of H of samples of Examples 1-6 which contain Eudragit E 100 or carboxymethylethylcellul ose remained almost unchanged after one-month storage, which suggests that the non-crystalline state of the samples was well maintained during storage. In the above experiment, the polarization of Example 5 and Reference 3 was measured by using spray dried samples to eliminate the influences of polarization caused by crystalline cellulose and magnesium stearate.

Table 1

| | Analysis articles | at the beginning of storage | 60° C, Airtight, 1 month | 40° C/75RH. 1 month |
|---|---|---|---|---|
| Example 1 | | | | |
| 10/5 | Polarization | none | none | none |
| | ΔH | 16.1 | 15.9 | 16.0 |
| 10/2 | Polarization | none | none | none |
| | ΔH | 15.5 | 15.7 | 15.4 |
| 10/1 | Polarization | none | none | none |
| | ΔH | 15.9 | 15.8 | 16.0 |
| 10/0.5 | Polarization | none | none | none |
| | ΔH | 16.2 | 16.0 | 15.9 |
| Reference 1 | Polarization | none | present | present |
| | ΔH | 14.5 | 3.1 | 4.8 |
| Example 2 | ΔH | 17.0 | 16.8 | 16.0 |
| Reference 2 | ΔH | 15.0 | 9.5 | 10.1 |
| Example 3 | ΔH | 17.5 | 16.9 | 16.0 |
| Example 4 | ΔH | 17.0 | 16.8 | 16.9 |
| Example 5 | | | | |
| 10/5 | Polarization | none | none | none |
| | ΔH | 13.7 | 13.2 | 13.2 |
| 10/2 | Polarization | none | none | none |
| | ΔH | 13.5 | 13.3 | 13.2 |
| 10/1 | Polarization | none | none | none |
| | ΔH | 13.9 | 13.8 | 13.4 |
| 10/0.5 | Polarization | none | none | none |
| | ΔH | 13.1 | 12.9 | 12.9 |
| Reference 3 | Polarization | none | present | present |
| | ΔH | 12.8 | 5.0 | 6.0 |
| Example 6 | ΔH | 14.1 | 13.6 | 14.7 |
| | | | (mcal/mg) | |

* Reference 1: prepared by repeating the procedure of Example 1 with the excerption that there was no Eudragit E 100. In the table, 10/5 and the like represent the ratio by weight of the antibiotic/Eudrag it E 100.

** Reference 2: prepared by repeating the procedure of Example 2 with the excerption that there was no Eudragit E 100.

***Reference 3: prepared by repeating the procedure of Example 5 with the excerption that there was no carboxymethylcellulose (CMEC). 10/5 and the like represent the ratio by weight of the antibiotic/CMEC.

Experiment 3

X-Ray Diffraction

The X-ray diffractions of the above-obtained samples were measured by using a JDX-8030 X-ray diffraction apparatus (JEOL LTD.) through a Ni-filter with a CuKα x-ray (40KV, 20mA) at a scanning speed of 1.2° /min. (Fig. 1). High X-ray diffraction peaks were observed with the crystalline powders, while no X-ray dif fraction peaks were observed with the samples of Example 1 (10/2) and Reference 1. After the storage for one month under airtight conditions at 60 °C, no diffraction peaks was observed with the

sample of Example 1 (10/2), while diffraction peaks representing the crystalline state were observed with the sample of Reference 1 after storage for 7 days, which revealed the transition of the sample to crystalline state.

Experiment 4

Solubility Test

Solubility of the samples was determined by using a USPXXI dissolution apparatus (Toyama Sangyo Co., LTD.) and 200 ml of buffer (adjusted at pH 4.0, prepared by mixing JP dissolution test solution 1 and JP dissolution test solution II) at 37 ± 0.5° C with stirring at 100 rpm paddle rotation. 250 mg (potency equivalent) of sample was added to the test solution, and the solubility of the sample was determined by high performance liquid chromatography under time course investigation. The solubilities of the crystalline samples were no more than about a few micrograms per ml, while the solubility of the non-crystalline sample (Reference 3) which was prepared by removing solvent from the solution of the crystalline sample in an organic solvent was 725μg/ml after 5 minutes (Fig. 2). However, the solubility decreased immediately after about 5 minutes because of crystallization of the antibiotic. On the other hand, the solubility of the capsules of Example 2 gradually increased and the capsules were completely dissolved in the buffer within 60 minutes. After storage for one month at 60° C , the solubility of the granules of Reference 3 was no more than 240μg/ml after five minutes, and became the same as that of the crystalline powders after 60 minutes. The solubility of the capsule of Example 2 remained unchanged after storage under the same conditions, which suggests that the pharmaceutical composition of the present invention is stable (Fig. 2).

After the tablets of Example 3 and Example 4 were stored for one month at 60° C, the solubility of the tablet of Example 4 remained unchanged and the tablet was well soluble in the buffer (Fig. 3). The solubility of the tablet of Example 3 decreased slightly after storage, however, the tablet was still more stable than the sample of Reference 3. The solubility and rate of dissolution of the powders, prepared by dissolving carboxymethylethylcellulose (CMEC) and the antibiotic simultaneously in a mixture of methylene chloride and ethanol (1:1) and then removing the solvent by spray drying, decreased inversely with the increasing amount of carboxymethylethylce llulose: However, the powders were stable and did not show an decrease in solubility as was observed with the samples of Reference 3 (Fig. 4).

After the granules of Example 5 were stored for one month at 600C1 the solubility of the granules remained unchanged (Fig. 5). The solubility of the tablet of Example 6, like that of Example 5, remained unchanged after stored at 60 ° C for one month (Fig. 6).

Experiment 5

Blood level

50 mg equivalent/Kg of the samples of the antibiotic were administrated orally to four male dogs (beagle, weight of from 8 to 10 Kg) which were fasted overnight before the oral administration, and the blood levels of the antibiotic were determined. Blood was sampled at intervals of 0.5, 1.0, 2.0, 3.0, 4.0, 5.0, 6.0 and 8.0 hours after the administration, and the concentration of the antibiotic in blood plasma was determined by a bioassay procedure. The tablets of Example 4 were used for the experiment and, as a reference, capsules filled with the mixture of crystalline antibiotic (not larger than 200 mesh) and avicell (1:1) were administered in the same manner. The results are summarized in Fig. 7. The blood level was not detectable for one of the dogs to which the crystalline powders were administered. The maximum blood level (Cmax) and the corresponding time (Tmax) were 1.4 μg/ml (1.5 hrs), 0.6 μg/ml (2 hrs), and 0.4 μg/ml (2 hrs) for the other three dogs. On the other hand, Tmax of three dogs administered with the tablets of Example 4 was 2 hrs for each dog, and the corresponding Cmax of the dogs were 9.9, 8.4, and 7.2μg/ml. Cmax and Tmax of the other dog were 5.2μg/ml and 4 hours, respectively. AUCs (area under the curve, μg • hr/ml) are summarized in Table 2.

Table 2

| Dog | AUC (μ g • hr/ml) | |
|---|---|---|
| No. | Reference* | Example 4 |
| 1 | 3.98 | 40.1 |
| 2 | 1.48 | 26.8 |
| 3 | 1.61 | 32.9 |
| 4 | N.D.** | 28.6 |

* Crystalline antibiotic
**Not detected

From the foregoing description, it is clearly understood that the antibiotic composition of the present invention is sufficiently stable and highly soluble even after a prolonged storage, and has an excellent "delivery" when administered orally.

One of ordinary skill in the art will recognize that departures from and improvements to the disclosed preferred embodiments may be carried out while remaining within the scope of the present invention as recited in the appended claims.

**Claims**

1. An antibiotic composition comprising a dispersoid of a substantially non-crystalline 4″-O-(para-methoxyphenylacethyl)tylosin antibiotic dispersed in a base material selected from the group consisting of acrylic copolymers and cellulose polymer derivatives.
2. The antibiotic composition according to claim 1, wherein the ratio of the base material to the antibiotic is from 0.02 to 3 parts by weight of the base material to 1 part by weight of the antibiotic.
3. The antibiotic composition according to claim 1, wherein the acrylic copolymer is selected from the group consisting of methacrylic acid/methacrylate copolymer, methacrylate/methacrylate copolymer, methacrylic acid/methacrylate/aminoalkylmethacrylate copolymer, methacrylate/methacrylate/aminoalkylmethacrylate copolymer, and mixtures thereof.
4. The antibiotic composition according to claim 1, wherein the cellulose polymer derivative is selected from the group consisting of corboxymethylethylcellulose, cellulose phthalate derivatives, cellulose acetate succinate derivatives, and mixture thereof.
5. The antibiotic composition according to claim 1, wherein the dispersoid is prepared by dissloving the 4″-O-(para-methoxyphenylacet yl)tylosin antibiotic and a base material in a solvent, and removing the solvent under reduced pressure.
6. The antibiotic composition according to claim 1, wherein the dispersoid is prepared by dissloving the 4″-O-(para-methoxyphenylacetyl)tylosin antibiotic and a base material in a solvent, and removing the solvent by a spray drying process.
7. A method for preparing an antibiotic composition comprising the steps of dissolving 4″-O-(para-methoxyphenylacethyl)tylosin antibiotic and a base material selected from the group consisting of acrylic copolymers and cellulose polymer derivatives in a solvent, and removing the solvent.
8. The method for preparing an antibiotic composition according to claim 7, wherein the solvent is removed under a reduced pressure.
9. The method for preparing an antibiotic composition according to claim 7, wherein the solvent is removed by a spray drying process.
10. A pharmaceutical composition for the treatment of an infectious disease, comprising:
a dispersoid of substantially noncrystalline 4″-O-(para-methoxyphenylacetyl)tylosin antibiotic dispersed in a base material selected from the group consisting of acrylic copolymers and cellulose polymer derivatives; and a pharmaceutically acceptable carrier or coating.
11. A method for the treatment of an infectious disease comprising the step of administering to a mammal an effective amount of an antibiotic composition comprising a dispersoid of a substantially non-crystalline 4″-O-(para-methoxyphenylacetyl)tylosin antibiotic dispersed in a base material selected from the group consisting of acrylic copolymers and cellulose polymer derivatives.
12. A method according to claim 11, wherein said step of administration is performed on a human being.

FIG. 1

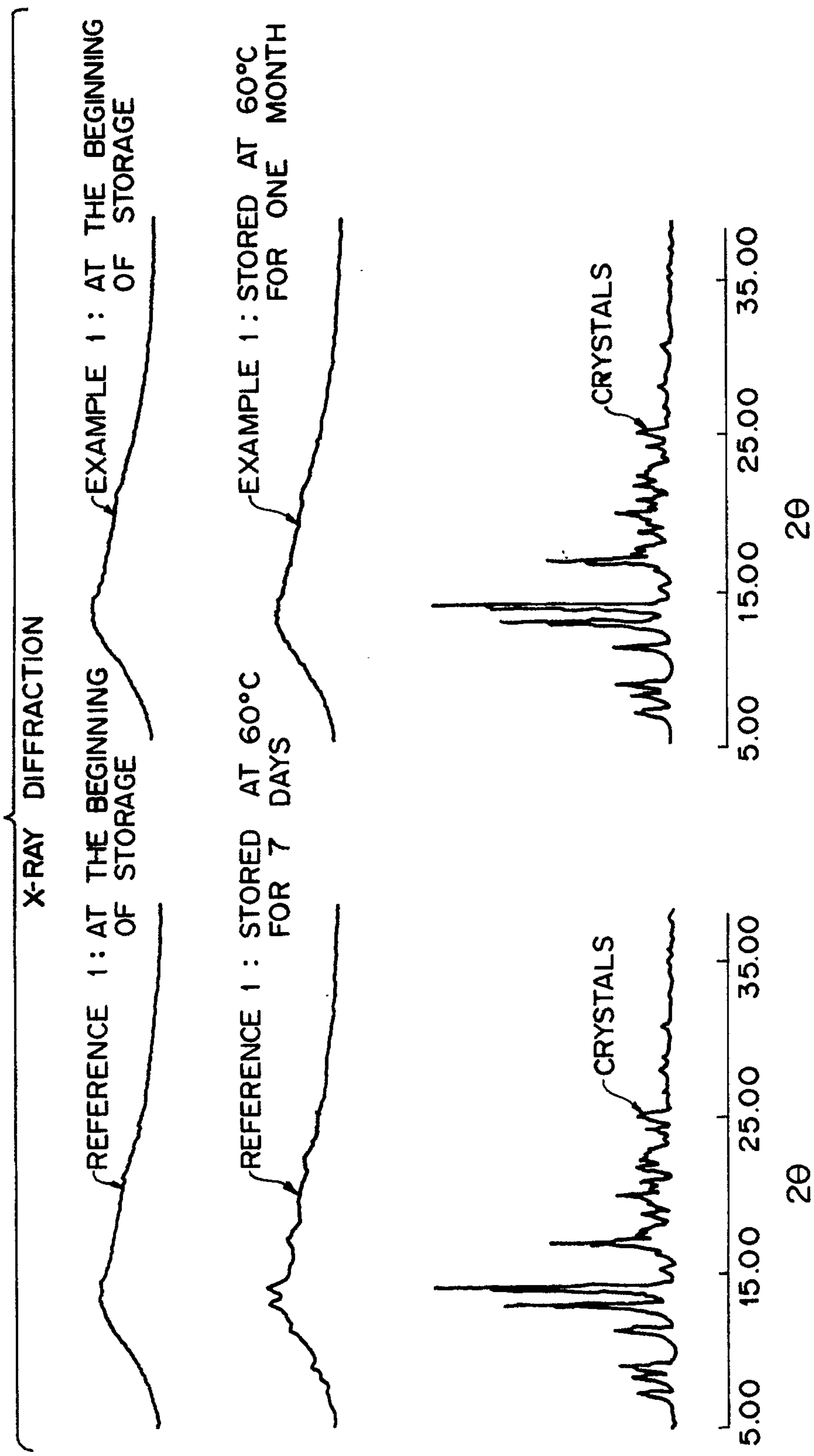
X-RAY DIFFRACTION
REFERENCE 1 : AT THE BEGINNING OF STORAGE
REFERENCE 1 : STORED AT 60°C FOR 7 DAYS
CRYSTALS
5.00
15.00
25.00
35.00
2θ
EXAMPLE 1 : AT THE BEGINNING OF STORAGE
EXAMPLE 1 : STORED AT 60°C FOR ONE MONTH
CRYSTALS
5.00
15.00
25.00
35.00
2θ

# FIG. 2

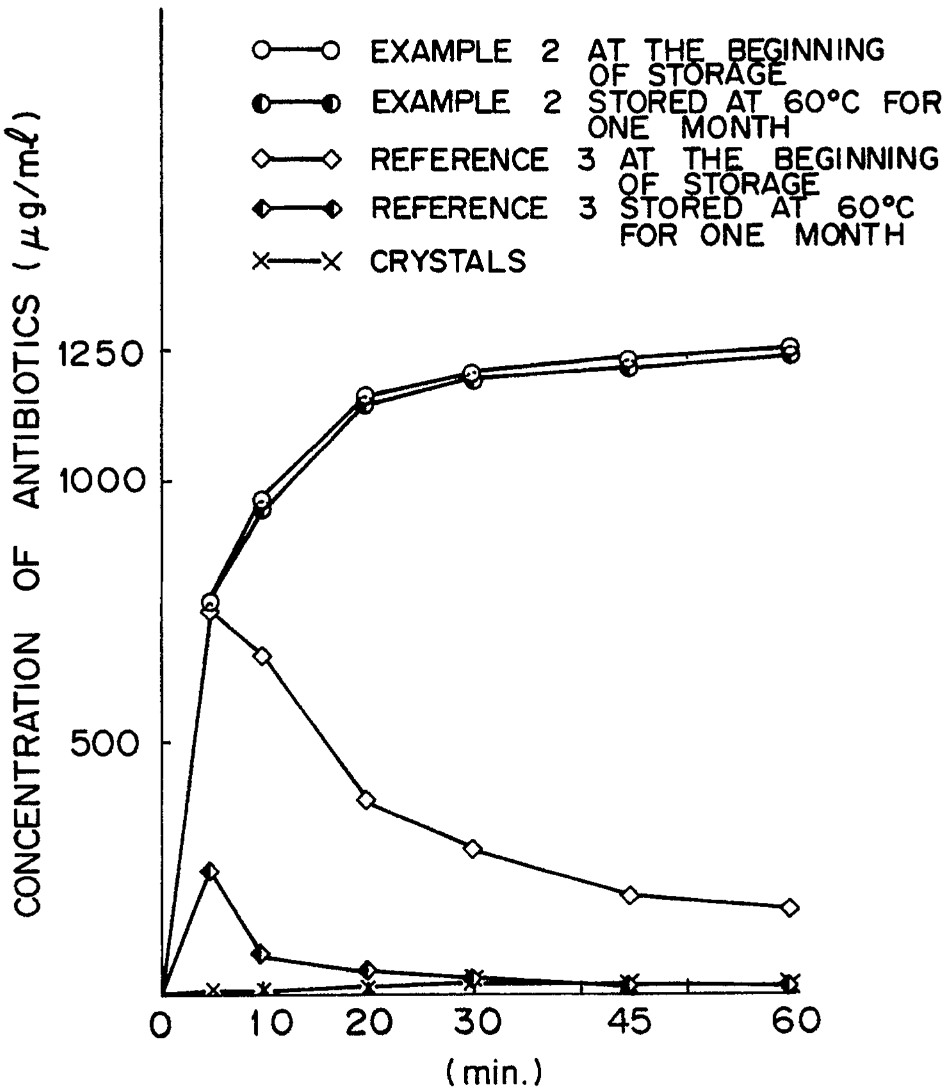
EXAMPLE 2 AT THE BEGINNING OF STORAGE
EXAMPLE 2 STORED AT 60°C FOR ONE MONTH
REFERENCE 3 AT THE BEGINNING OF STORAGE
REFERENCE 3 STORED AT 60°C FOR ONE MONTH
CRYSTALS
CONCENTRATION OF ANTIBIOTICS (μg/mℓ)
1250
1000
500
0
10
20
30
45
60
(min.)

FIG. 3

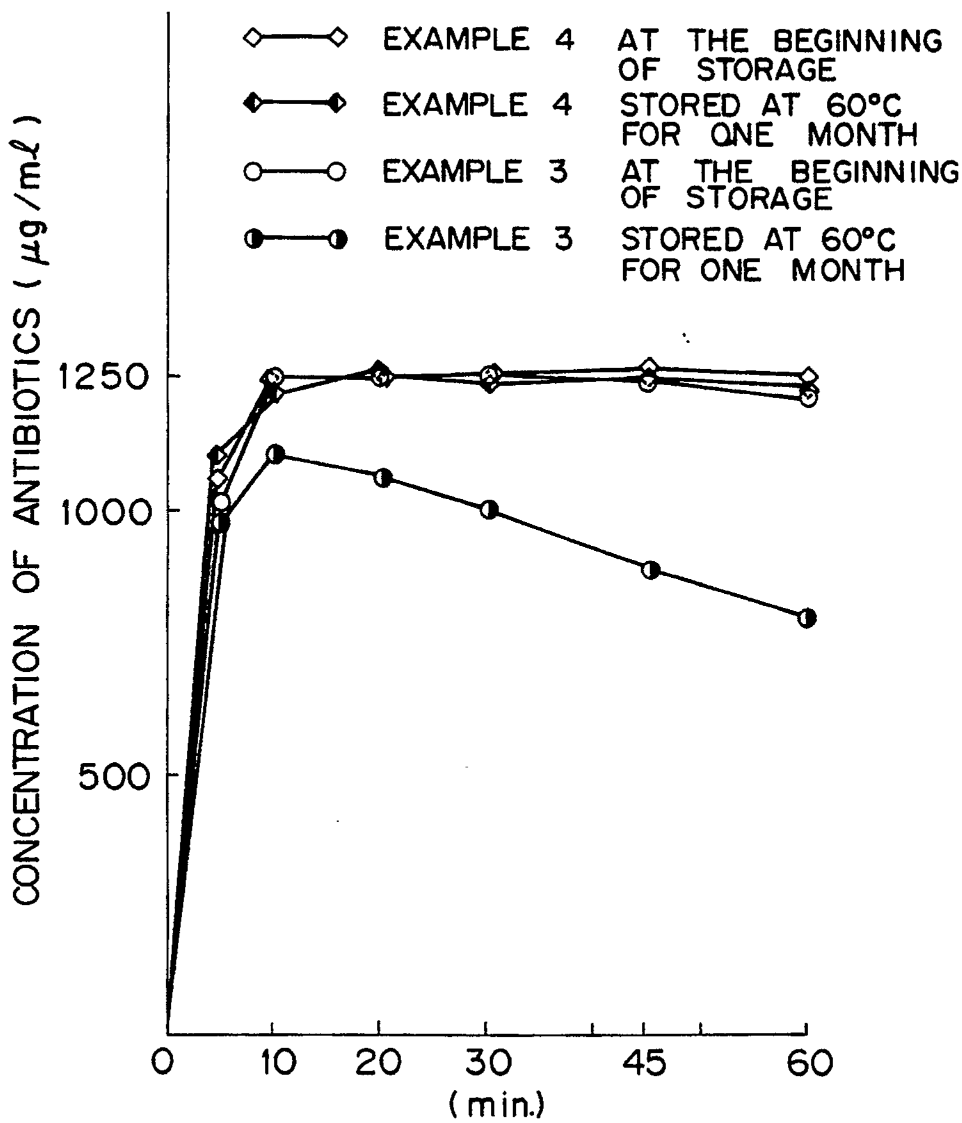
EXAMPLE 4 AT THE BEGINNING OF STORAGE
EXAMPLE 4 STORED AT 60°C FOR ONE MONTH
EXAMPLE 3 AT THE BEGINNING OF STORAGE
EXAMPLE 3 STORED AT 60°C FOR ONE MONTH
CONCENTRATION OF ANTIBIOTICS ( μg/mℓ )
1250
1000
500
0
10
20
30
45
60
( min.)

FIG. 4

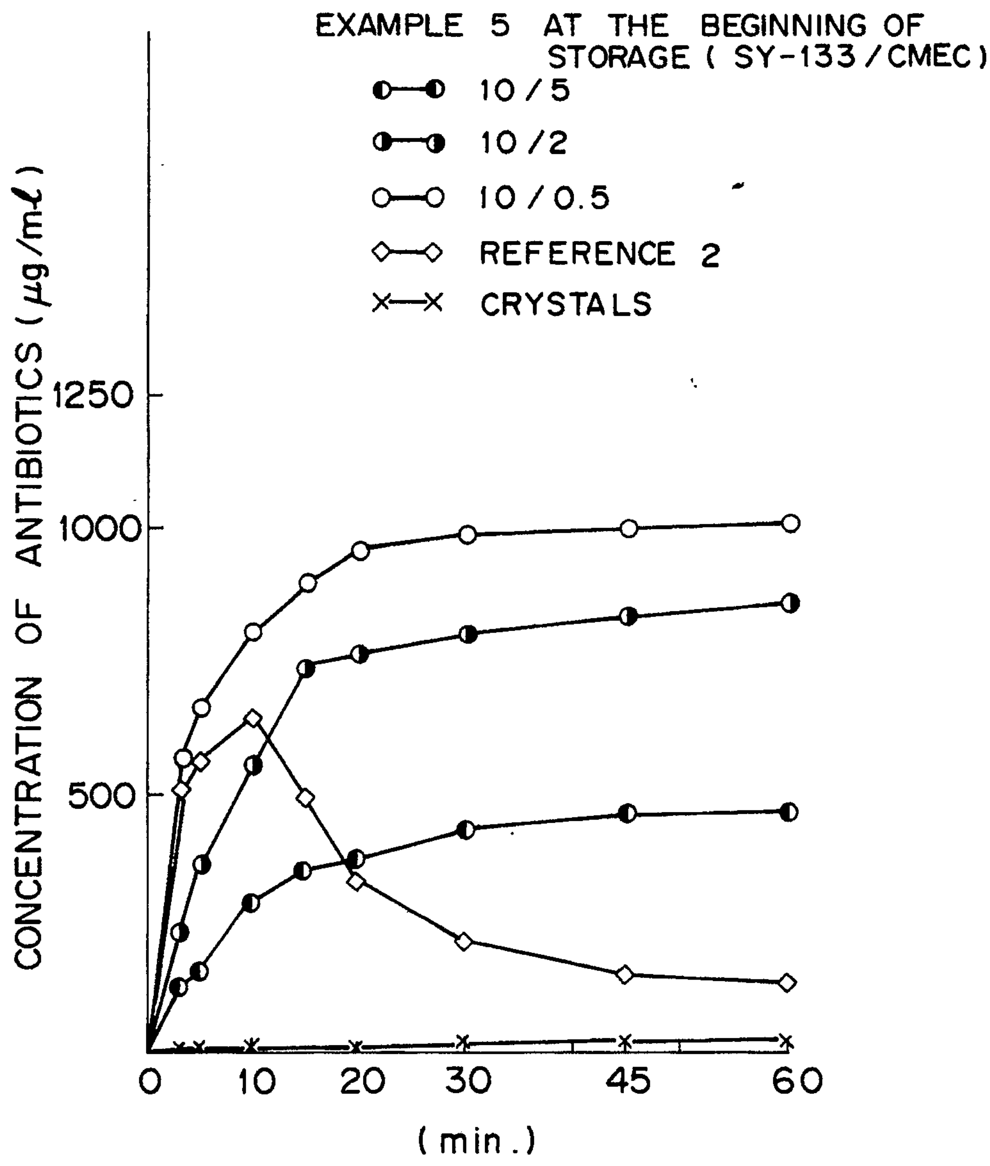
EXAMPLE 5 AT THE BEGINNING OF STORAGE ( SY-133 / CMEC )
10 / 5
10 / 2
10 / 0.5
REFERENCE 2
CRYSTALS
CONCENTRATION OF ANTIBIOTICS ( μg/mℓ )
1250
1000
500
0
10
20
30
45
60
( min. )

FIG. 5

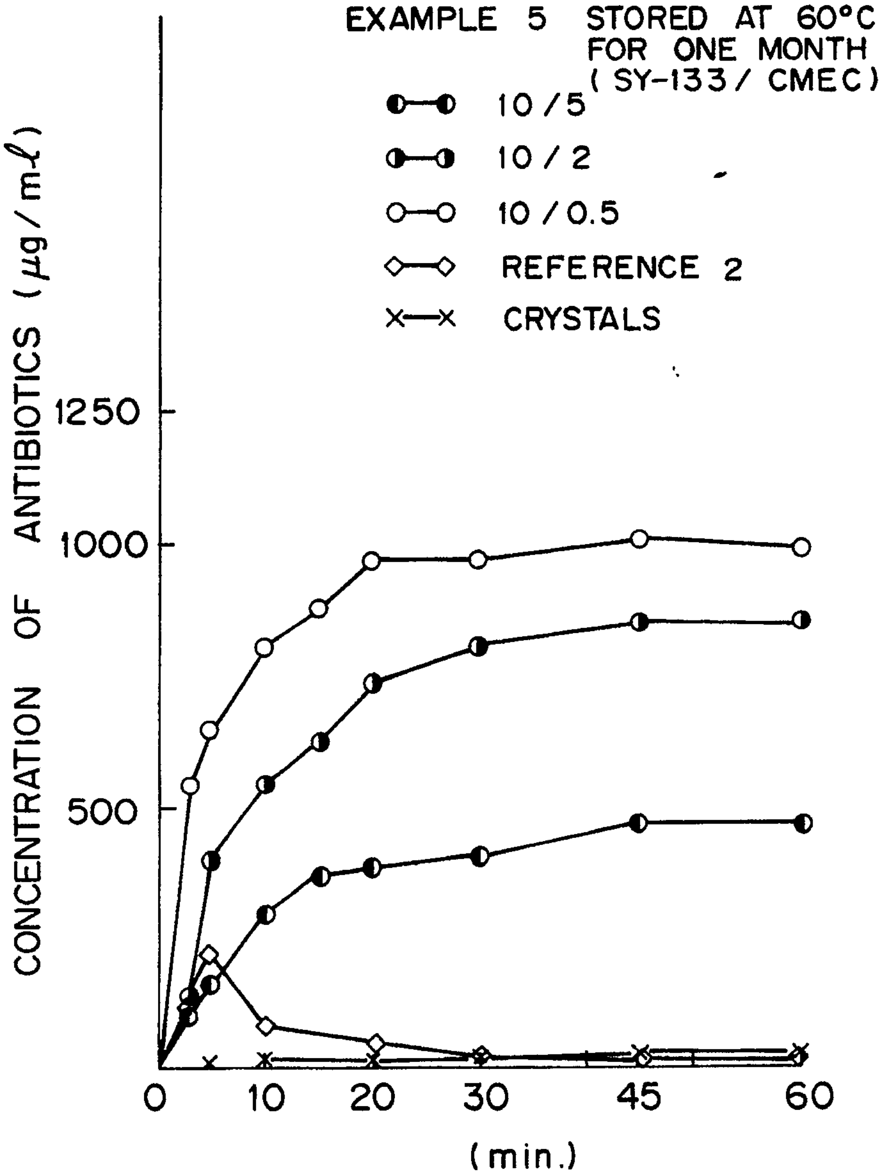
EXAMPLE 5 STORED AT 60°C
FOR ONE MONTH
( SY-133 / CMEC )
10 / 5
10 / 2
10 / 0.5
REFERENCE 2
CRYSTALS
CONCENTRATION OF ANTIBIOTICS ( μg / mℓ )
1250
1000
500
0
10
20
30
45
60
( min.)

FIG.6

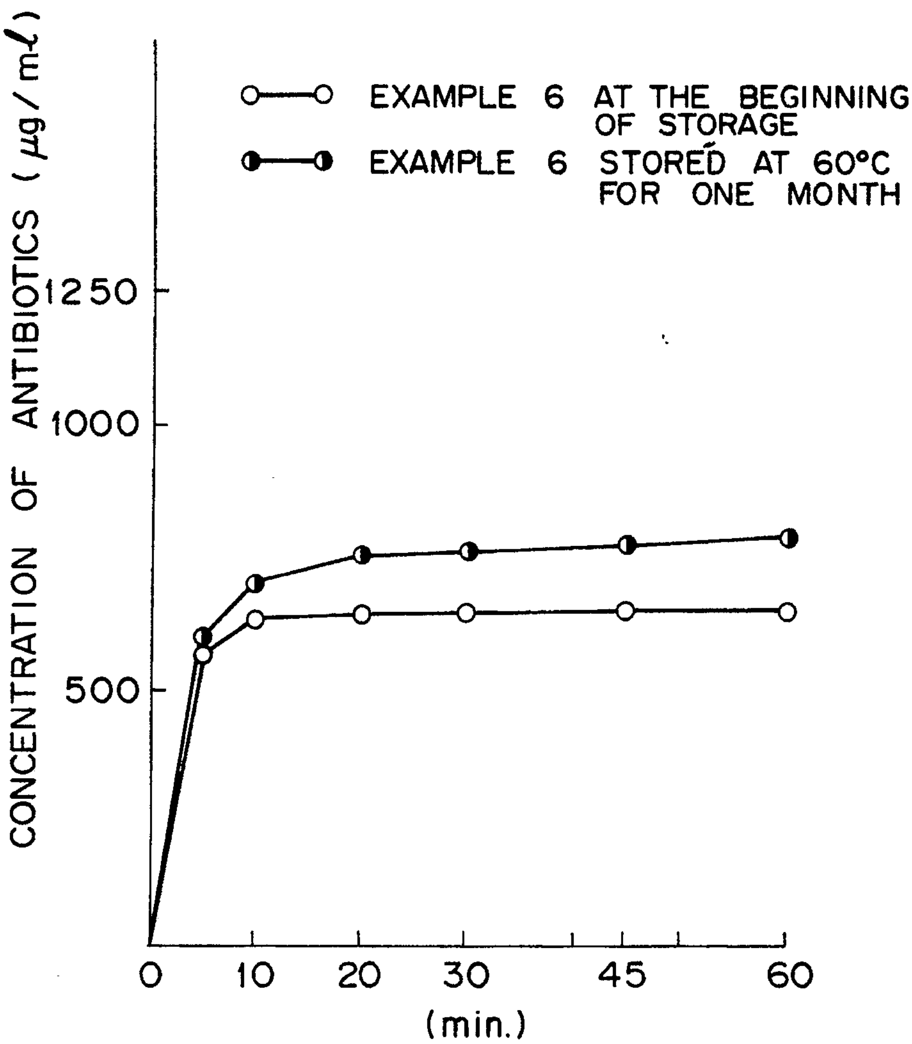
EXAMPLE 6 AT THE BEGINNING OF STORAGE
EXAMPLE 6 STORED AT 60°C FOR ONE MONTH
CONCENTRATION OF ANTIBIOTICS ( μg / mℓ )
1250
1000
500
0
10
20
30
45
60
(min.)

FIG.7

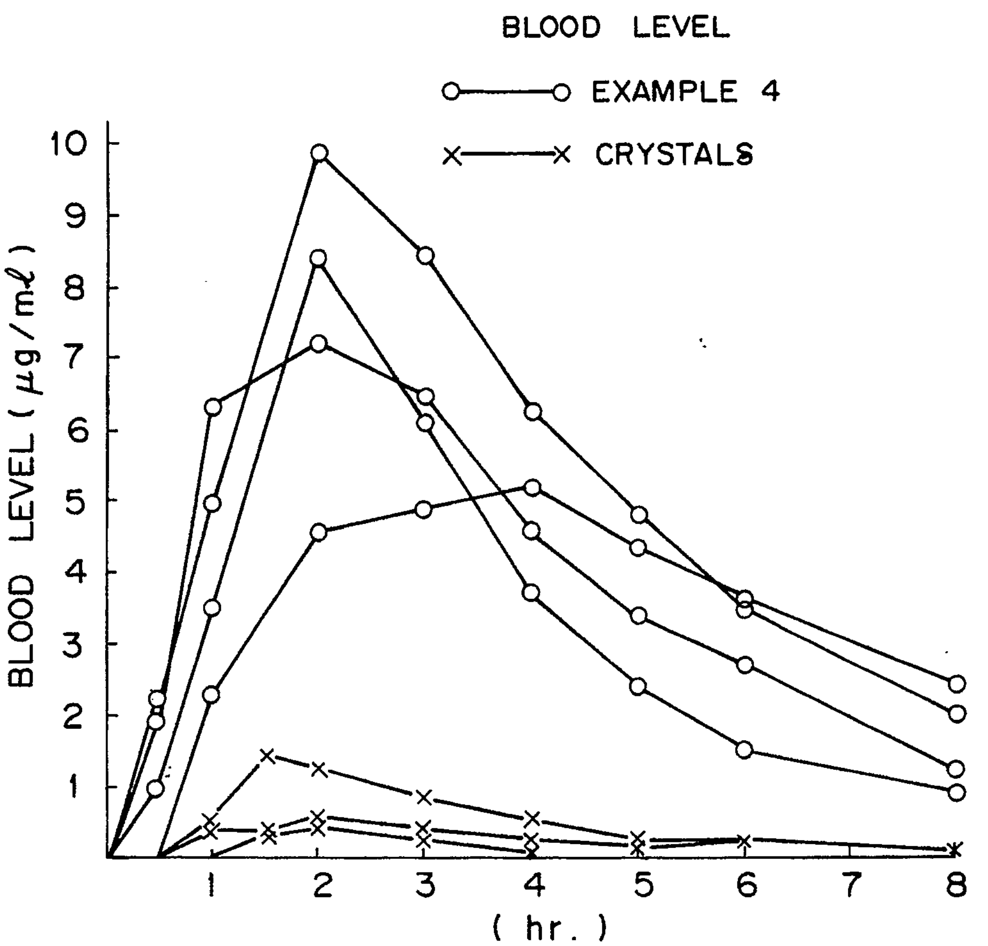
BLOOD LEVEL
EXAMPLE 4
CRYSTALS
BLOOD LEVEL (μg/mℓ)
10
9
8
7
6
5
4
3
2
1
1
2
3
4
5
6
7
8
(hr.)

European Patent Office

# PARTIAL EUROPEAN SEARCH REPORT

which under Rule 45 of the European Patent Convention shall be considered, for the purposes of subsequent proceedings, as the European search report

Application number

EP 90 11 5553

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 025 698 (ELI LILLY)<br>* Claims 1,3-4,16,18-19; page 9, lines 28-30; page 10, lines 1-4 *<br>-- | 1,6,9 | A 61 K 31/71<br>A 61 K 9/16<br>A 61 K 9/18<br>A 61 K 9/20<br>A 61 K 9/48<br>A 61 K 9/50 |
| D,A | CHEM. PHARM. BULL., vol. 29, no. 9, 1981, pages 2675-2682; T. SATO et al.: "Difference in physico-pharmaceutical properties between crystalline and noncrystaline 9,3"-diacetylmidecamycin"<br>* Page 2676: Formula; page 2677, lines 4-7 *<br>---- | 1,6,9 | |
| | | | TECHNICAL FIELDS SEARCHED (Int. Cl.4) |
| | | | A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10

Claims searched incompletely:

Claims not searched: 11-12

Reason for the limitation of the search:

Method for treatment of the human or animal body by surgery or therapy (See art. 52(4) of the European Patent Convention)

| Place of search | Date of completion of the search | Examiner |
|---|---|---|
| THE HAGUE | 23-11-1990 | SCARPONI |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82